# EUROPEAN PATENT APPLICATION

(11) **EP 4 752 211 A1**
(43) Date of publication of application: **03.06.2026**
(21) Application number: 25219381.8
(22) Date of filing: 28.11.2025
(51) Int. Cl.: C12M 1/34, C12M 3/00, G01N 21/31, G01N 21/61, G01N 21/3504

(54) **SYSTEM AND METHOD FOR DETERMINING CELLULAR RESPIRATION AND NUTRIENT METABOLISM OF LIVING CELLS OR TISSUES**

(30) Priority: 29.11.2024 DE 102024135470
(71) Applicant: Technische Universität Dortmund, Körperschaft des öffentlichen Rechts, 44227 Dortmund (DE); Universität zu Köln, Körperschaft des öffentlichen Rechts, 50923 Köln (DE)
(72) Inventor: Schütz, Natalie, 54295 Trier (DE); Zimmer, Moritz, 54295 Trier (DE); Ortiz Perez, Alvaro, 44139 Dortmund (DE); Rodriguez Gutierrez, Gabriel, 44379 Dortmund (DE); Palzer, Stefan, 44227 Dortmund (DE); Kreuzaler, Peter, 50670 Köln (DE)
(74) Representative: Michalski Hüttermann & Partner Patentanwälte mbB

(57) **Abstract**

The invention relates to a system (1) for determining cellular respiration and nutrient metabolism of living cells or tissues, the system comprising:
a cell container (2) configured to receive and maintain a biological sample (3),
a gas volume (4) in fluid communication with the cell container (2), the gas volume (4) containing gaseous components produced by metabolic activity of the biological sample (3),
an optical detection unit (5) configured to detect a target gas species in the gas volume by wavelength-selective absorption spectroscopy, the optical detection unit being further configured to distinguish between different isotopic species of the target gas species, the optical detection unit (5) comprising an illumination source (6) and detector arrangement (7) optically coupled to the optical detection unit (6) and configured for measurement of absorption signals corresponding to different isotopes of the target gas species, and
an electronic control and evaluation unit (8) operatively connected to the optical detection unit (7), the electronic control and evaluation unit (8) being configured to acquire and process absorption data, determine absolute values and/or temporal variations of the target gas species concentration and isotopic composition, and output respiration-related parameters of the biological sample (3).

In this way, a system is provided that enables precise and efficient determination of carbon dioxide produced by living cells or tissues under physiological conditions.

## Description

The present invention relates to the field of physiological and biochemical analysis of living cells and tissues, in particular to methods and systems for determining cellular respiration and nutrient metabolism in higher organisms. Cellular respiration represents one of the most fundamental biochemical processes of life, in which organic molecules such as carbohydrates, proteins, and lipids are metabolized to generate energy in the form of adenosine triphosphate (ATP). During this process, carbon dioxide (CO₂) is produced as the final product of oxidative metabolism. Quantitative and temporal analysis of this CO₂ production provides valuable insight into the metabolic state and vitality of cells.

In the life sciences, accurate assessment of cellular respiration and nutrient utilization is essential for a wide range of applications. In medical research, it contributes to the understanding of metabolic disorders, mitochondrial dysfunction, and tumor metabolism. In pharmaceutical research, monitoring of cellular respiration is used to evaluate the effects of drugs or toxic compounds on cellular energy production. In biotechnology and tissue engineering, measurement of oxygen consumption and carbon dioxide evolution serves as an indicator of cell proliferation, differentiation, and viability in culture systems.

Conventional methods for analyzing respiration and metabolism often rely on indirect measurement techniques. These include monitoring of oxygen concentration changes in the medium, calorimetric detection of metabolic heat, or analysis of extracellular metabolites. Although such approaches provide useful information, they typically lack temporal resolution, require discontinuous sampling, or are limited in sensitivity when applied to miniature or microfluidic systems. Furthermore, the determination of CO₂ as a metabolic end product is often performed ex situ, requiring complex gas-handling procedures that may disturb the biological system under investigation.

In recent years, there has been increasing demand for analytical methods capable of continuously and non-invasively monitoring metabolic activity at the cellular or subcellular level. This demand is driven in part by the growing importance of preclinical research on tumor-specific metabolism, where malignant cells exhibit characteristic alterations in respiratory and biosynthetic pathways. The ability to dynamically observe such processes under physiological conditions and in real time is crucial for understanding disease mechanisms and for developing targeted therapeutic strategies.

Despite substantial advances in biosensing and microanalytical technologies, there remains a lack of compact and reliable systems that can directly quantify metabolic gas exchange with high temporal resolution and without perturbing the cell environment. The technical challenges arise from the need to detect very small changes in gas composition in confined volumes, to achieve precise differentiation between isotopic species of carbon dioxide, and to integrate such measurements into standard cell culture workflows.

It is the object of the invention to provide a system and a method that enables precise and efficient determination of a target gas species produced by living cells or tissues under physiological conditions.

This object is addressed by the subject matter of the independent claims. Embodiments of the invention are defined in the dependent claims.

Therefore, according to the invention, a system for determining cellular respiration and nutrient metabolism of living cells or tissues is provided, the system comprising:
a cell container configured to receive and maintain a biological sample under controlled environmental conditions,
a gas volume in fluid communication with the cell container, the gas volume containing gaseous components produced by metabolic activity of the sample,
an optical detection unit configured to detect a target gas species in the gas volume by wavelength-selective absorption spectroscopy, the optical detection unit being further configured to distinguish between different isotopic species of the target gas species, the optical detection unit comprising an illumination source and detector arrangement optically coupled to the optical detection unit and configured for measurement of absorption signals corresponding to the different isotopes of the target gas species, and
an electronic control and evaluation unit operatively connected to the optical detection unit, the electronic control and evaluation unit being configured to acquire and process absorption data, determine absolute values and/or temporal variations of the target gas species concentration and isotopic composition, and output respiration-related parameters of the biological sample.

By combining the cell container, gas volume, optical detection unit, control electronics, and communication interface in a single system, continuous observation of metabolic gas exchange may be carried out without disturbing the biological sample. The system may provide time-resolved information on cellular respiration and nutrient metabolism and may be suited for applications in biomedical research, pharmacological testing, and biotechnology.

The optical detection unit may be arranged such that only light interacts with the gas volume while all structural components of the unit remain physically separated from the interior of the gas space. In this configuration, the illumination source, detector, mirrors, and optical mounts can be positioned outside the sealed gas volume, with optical access provided solely through transparent windows. This separation prevents any hardware element from coming into contact with the gas phase and avoids introduction of contaminants or particles into the measurement environment. It also facilitates cleaning and sterilization of the gas-handling components, since only the surfaces of the cell container and the gas volume require treatment, whereas the optical hardware does not need to be exposed to sterilizing agents or elevated temperatures. This arrangement supports reliable long-term use under laboratory or incubation conditions while maintaining the integrity of both the biological sample and the measurement optics.

The cell container may be configured to receive and maintain a biological sample under controlled environmental conditions. This may allow the biological sample, such as cells, tissues, or organoids, to remain viable and metabolically active during measurement. Stable conditions of temperature, humidity, and gas composition may ensure that metabolic processes proceed in a reproducible manner, so that variations in the detected target gas species concentration can be attributed to physiological activity rather than to environmental fluctuations. Furthermore, maintaining a controlled environment within the container may reduce contamination risk and may enable long-term or repeated measurements on the same sample without removal from the analytical system.

A communication interface may be provided which is configured to transmit the respiration-related parameters or derived data to an external device for display, storage or further analysis, i. e. to a display or to a computer.

In some embodiments, the electronic control and evaluation unit is configured to process absorption data in real time. In this context, real-time processing may refer to the acquisition, conversion, and computational evaluation of detector signals with a temporal resolution sufficient to follow changes in carbon dioxide concentration and isotopic composition as they occur during cellular metabolism. The control and evaluation unit may therefore include electronic components capable of high-speed data acquisition and processing. Such components may comprise analog-to-digital converters, signal amplifiers, and one or more processors or microcontrollers programmed to execute measurement routines and data analysis algorithms. The system may thus allow continuous or quasi-continuous recording of respiration-related parameters without requiring manual data post-processing.

Real-time processing of the absorption data may provide the possibility to display variations in metabolic activity immediately after their occurrence. This may be useful, for example, when studying transient responses of cells to changing environmental conditions or to the administration of nutrients, drugs, or inhibitors. By acquiring and evaluating the measurement data continuously, the system may further enable adaptive control of measurement parameters such as illumination intensity, integration time, or temperature regulation, thereby maintaining stable operating conditions. In certain implementations, the real-time capability may also support time-synchronized data exchange with external laboratory instruments or software platforms. For instance, respiration data may be transmitted to an external computing device via the communication interface and may be processed together with optical, electrical, or biochemical signals originating from the same biological sample. Such integration may allow correlation of metabolic gas exchange with other physiological indicators. Through the configuration for real-time processing, the system may thus provide improved temporal resolution and responsiveness in the observation of cellular metabolism.

In certain embodiments, the illumination source and detector arrangement is configured for non-dispersive infrared (NDIR) measurement of absorption signals corresponding to the different isotopes of the target gas species. In such a configuration, infrared radiation of one or more selected wavelength bands may be directed through a gas volume containing carbon dioxide, and the intensity of the transmitted or reflected radiation may be measured by the detector. Since the different isotopes of the target gas species exhibit characteristic absorption features at slightly different infrared wavelengths, measurement at these distinct wavelength regions may allow separate determination of their respective concentrations.

The NDIR principle may provide a direct and contact-free measurement of gaseous the target gas species without the need for chemical conversion or sampling of the gas phase. This may allow the biological sample to remain undisturbed during measurement. The approach may further permit time-resolved detection of variations in total target gas species concentration and in the isotopic composition of the target gas species produced by the biological sample. Information obtained in this way may be indicative of the metabolic activity or substrate utilization of the cells or tissues examined. The configuration for NDIR detection may enable compact implementation of the optical detection unit and may be compatible with long-term or continuous operation. Because NDIR detection relies on absorption of infrared radiation characteristic for the respective isotopic species, it may be suitable for distinguishing target gas species molecules originating from different labeled nutrient sources. This may be useful, for example, when isotopically labeled substrates are supplied to the cells to study nutrient conversion pathways.

In some embodiments, the optical detection unit comprises at least one optical path having an optical path length adapted for detection of target gas species concentrations in the range of less than 1 %, preferably less than 0.1 %. The optical path may form part of the gas volume or may be arranged adjacent to it, with optical windows defining a measurement path through which infrared radiation passes. The length of this path may be selected such that measurable absorption signals can be obtained even at the relatively low target gas species concentrations typically produced by cellular respiration in small volumes. By selecting an appropriate optical path length, the signal intensity detected by the infrared sensor may reach a level suitable for reliable data acquisition without requiring excessive amplification. The ability to detect concentrations in the sub-percent range may make the system suitable for measurements in confined gas volumes, for example within microfluidic chambers or sealed cell containers, where total gas production is limited. By configuring the optical detection unit in this way, the system may be capable of detecting small variations in target gas species concentration resulting from metabolic activity, thereby allowing sensitive and reproducible observation of cellular respiration even under low-volume or low-flux conditions.

In certain embodiments, the illumination source of the optical detection unit comprises a modulated infrared light-emitting diode (IR-LED) or a laser diode, and the detector comprises a wavelength-selective photodetector or a thermal detector. The use of an infrared light-emitting or laser diode as the illumination source may allow generation of radiation at specific wavelength regions corresponding to absorption bands of carbon dioxide. The emitted radiation may be directed through the optical path toward the detector, and its intensity after passage through the gas volume may be recorded as a measure of absorption. Modulation of the radiation intensity or emission timing may be employed so that the detector can distinguish the measurement signal from background radiation or low-frequency drift. Such modulation may be achieved, for example, by periodically switching the diode current or by applying a defined modulation frequency under control of the electronic evaluation unit.

A wavelength-selective photodetector or thermal detector may be configured to receive the transmitted infrared radiation and convert it into an electrical signal proportional to its intensity. In some embodiments, the detector may include an optical filter or integrated resonant structure that defines the wavelength region to which it responds. This configuration may allow discrimination between absorption signals of different isotopic species, such as ¹²CO₂ and ¹³CO₂ when detecting CO₂ as the target gas species, by directing the detector to the corresponding spectral range. The combination of a diode-based illumination source and a wavelength-selective detector may permit compact and energy-efficient construction of the detection unit. Semiconductor-based emitters and detectors may exhibit long operational lifetimes and may not require mechanical alignment or moving optical parts. As a result, the optical assembly may be suited for continuous or long-term operation under laboratory or incubation conditions. The use of a modulated source together with a responsive detector may further enable signal-processing techniques such as synchronous detection or phase-sensitive amplification. These techniques may increase measurement sensitivity and may reduce the influence of ambient light or thermal fluctuations. The detector signal obtained in this way may be transmitted to the electronic control and evaluation unit, where it may be processed to determine target gas species concentration and isotopic composition over time.

In some embodiments, the optical detection unit is configured to measure absorption signals at at least two discrete wavelength bands corresponding to the absorption maxima of the different isotopes of the target gas species. Carbon dioxide molecules containing different carbon isotopes exhibit slightly shifted absorption features in the infrared region due to their differing molecular masses. By directing infrared radiation to two separate wavelength regions that coincide with characteristic absorption maxima of the different isotopes of the target gas species, the optical detection unit may distinguish the isotopic composition of the target gas species present in the gas volume. For this purpose, the detection unit may include optical elements that define or select the respective wavelength bands. These elements may comprise optical filters, coatings, or other wavelength-selective components that transmit or detect radiation in narrowly defined spectral ranges. The optical system may therefore be capable of registering two independent absorption signals that correspond to the two isotopic species.

The measurement at two discrete wavelength bands may allow the electronic control and evaluation unit to determine both the total target gas species concentration and the relative contribution of an isotope of the target gas species. From the ratio of these signals, an isotopic fraction or enrichment value may be derived. This information may provide insight into the metabolic utilization of isotopically labeled substrates introduced into the biological sample, such as ¹³C-labeled glucose or glutamine. Recording absorption at multiple discrete wavelengths may also allow internal referencing of the measurement, which may reduce sensitivity to variations in optical intensity or temperature drift. By comparing the two absorption signals, systematic influences that affect both channels similarly may be compensated, potentially improving the reproducibility of the measurement.

In some embodiments, the electronic control and evaluation unit comprises a microcontroller or microprocessor that is configured to demodulate the detector signal and compute the ratio between the different isotopes of the target gas species concentrations. The detector of the optical detection unit may generate an electrical signal whose amplitude or phase depends on the intensity of the transmitted or reflected infrared radiation. When the illumination source is operated in a modulated manner, the detector signal may contain a corresponding modulation component that represents the absorption behavior of the gas volume. The microcontroller or microprocessor may be programmed to extract this component from the raw signal by digital or analog demodulation. Such demodulation may be carried out by correlation with a reference signal derived from the modulation of the illumination source.

After demodulation, the resulting signal values may be converted into quantities proportional to the absorption at the selected wavelength bands for the different isotopes of the target gas species, respectively. The microcontroller or microprocessor may then calculate the ratio of the two absorption signals, which may correspond to the relative concentrations of the isotopic species in the gas volume. The ratio may be further processed to derive respiration-related parameters, such as isotopic enrichment or substrate utilization. The use of a microcontroller or microprocessor may allow the system to carry out these computations automatically and with high temporal resolution. The implementation of digital signal processing routines may reduce the influence of electrical noise and may provide stable operation even under varying measurement conditions. In certain embodiments, the microcontroller may also control additional system components such as illumination modulation, temperature stabilization, or data communication, thereby coordinating the measurement process as a whole.

In some embodiments, the gas volume is hermetically sealed with respect to the ambient environment. The gas volume may be formed as a defined chamber or headspace that communicates with the interior of the cell container. Its enclosure may be constructed such that uncontrolled leakage of gases to the environment is avoided. This sealing may ensure that variations in the measured gas composition originate primarily from the metabolic activity of the biological sample rather than from ambient fluctuations. A hermetically sealed configuration may also protect the measurement process from changes in laboratory atmosphere, humidity, or background target gas species concentration.

The invention also relates to a method for determining cellular respiration and nutrient metabolism of living cells or tissues, the method comprising the steps of:
receiving and maintaining a biological sample under controlled environmental conditions in a cell container,
allowing gaseous components produced by metabolic activity of the sample to accumulate in a gas volume in fluid communication with the cell container,
illuminating the gas volume with infrared radiation and detecting absorption signals corresponding to different isotopes of a target gas species,
acquiring and processing absorption data to determine temporal variations of the target gas species concentration and isotopic composition, and
outputting respiration-related parameters of the biological sample and optionally transmitting the parameters or derived data for display, storage or further analysis.

Further embodiments, functions, and advantages of this method arise in analogy to the system described further above.

The invention will be explained in greater detail below with reference to the drawings on the basis of an embodiment of the invention.

In the drawings
- Fig. 1: schematically depicts system 1 for determining cellular respiration and nutrient metabolism of a biological sample according to an embodiment of the invention and
- Fig. 2: depicts a flow chart of a method according to an embodiment of the invention.

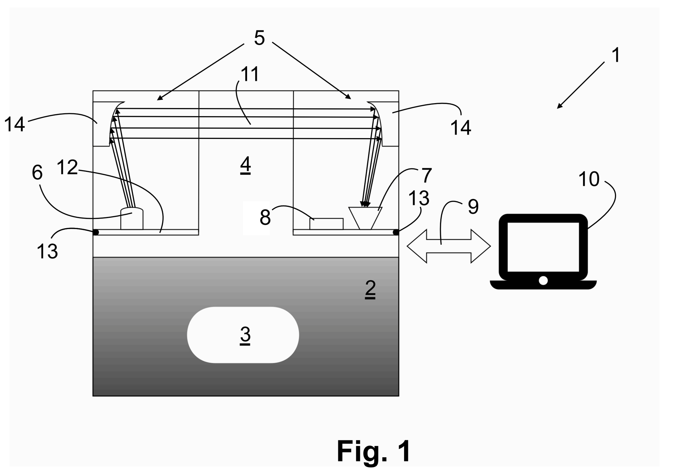

From Fig. 1, a system 1 for determining cellular respiration and nutrient metabolism of living cells or tissues according to an embodiment of the invention is shown. The system 1 is designed to enable direct and time-resolved determination of carbon dioxide produced by a biological sample 3 under physiological conditions, while maintaining the sample in an undisturbed and controlled environment.

The system 1 comprises a cell container 2, which is configured to receive and maintain a biological sample 3 such as living cells, tissues, or organoids. The cell container 2 may provide controlled environmental conditions, including defined temperature, humidity, and gas composition, so that the biological sample 3 remains viable and metabolically active during measurement. The maintenance of a stable environment ensures that variations in the detected CO₂ concentration (target gas species concentration) originate from metabolic activity rather than from external fluctuations. The cell container 2 is further arranged in fluid communication with a gas volume 4, which contains the gaseous components produced by the metabolic activity of the biological sample 3.

The gas volume 4 is hermetically sealed with respect to the ambient environment by sealings 13. This sealing prevents uncontrolled gas exchange with the surroundings and ensures that any change in gas composition detected during the measurement arises from the biological sample 3 itself. The sealed configuration also protects the measurement process from external variations in humidity or background CO₂ concentration and contributes to reproducible results.

An optical detection unit 5 is provided for detecting CO₂ in the gas volume 4 by wavelength-selective absorption spectroscopy. The optical detection unit 5 is further configured to distinguish between different isotopic species of CO₂, specifically ¹²CO₂ and ¹³CO₂. For this purpose, the optical detection unit 5 comprises an illumination source 6 and a detector arrangement 7 that are optically coupled to one another. Off-axis parabolic mirrors 14 are provided to direct and focus light emitted by the illumination source 6 through the optical path of the gas volume 4 along an optical path 11 toward the detector arrangement 7. The optical configuration with off-axis parabolic mirrors 14 enables efficient beam guidance and minimizes optical losses, thereby supporting high sensitivity in the detection of small concentration variations.

The illumination source 6 and the detector arrangement 7 are configured for non-dispersive infrared (NDIR) measurement of absorption signals corresponding to ¹²CO₂ and ¹³CO₂. In this measurement principle, infrared radiation of selected wavelength bands is guided through the gas volume 4, and the detector arrangement 7 records the intensity of the transmitted radiation. Because ¹²CO₂ and ¹³CO₂ exhibit slightly different absorption features in the infrared spectrum, measurement at two discrete wavelength regions enables separate determination of their respective concentrations and thus the isotopic composition of the generated CO₂. The NDIR principle provides a direct and contact-free measurement of gaseous CO₂ without the need for chemical conversion or gas sampling, thereby allowing continuous monitoring of metabolic gas production without disturbing the biological sample 3. The optical path length is adapted for the detection of CO₂ concentrations in the range of less than 1 %. This configuration allows the detection of the small quantities of CO₂ typically produced by cellular respiration in small or microfluidic volumes and enables sensitive and reproducible observation of metabolic activity.

The illumination source 6 is realized as a modulated infrared light-emitting diode (IR-LED), which emits radiation in a wavelength region corresponding to the absorption bands of CO₂. The modulation of the light intensity or emission timing allows the detector arrangement 7 to differentiate the measurement signal from background radiation and low-frequency drift. The detector arrangement 7 is realized as a wavelength-selective photodetector, which converts the transmitted infrared radiation into an electrical signal proportional to the radiation intensity. The detector arrangement 7 includes an optical filter that defines the wavelength region to which it responds, enabling discrimination between absorption signals of ¹²CO₂ and ¹³CO₂. The combination of a modulated IR-LED and a wavelength-selective photodetector provides a compact, energy-efficient, and mechanically robust measurement configuration suitable for long-term and continuous operation.

The optical detection unit 5 is configured to measure absorption signals at two discrete wavelength bands corresponding to the absorption maxima of ¹²CO₂ and ¹³CO₂. From the ratio of the two absorption signals, the electronic control and evaluation unit 8 computes the relative concentrations of the isotopic species and derives respiration-related parameters such as isotopic enrichment or substrate utilization. Recording absorption at two wavelength bands also allows internal referencing, which compensates for intensity variations or thermal drift and thereby increases measurement reproducibility.

The electronic control and evaluation unit 8 is arranged on a printed circuit board 12 and is operatively connected to the optical detection unit 5. The electronic control and evaluation unit 8 includes electronic circuits and a microcontroller configured to demodulate the detector signal and compute the ratio between ¹²CO₂ and ¹³CO₂ concentrations. The unit 8 acquires and processes the absorption data in real time, determines temporal variations of CO₂ concentration and isotopic composition, and outputs respiration-related parameters of the biological sample 3. Real-time processing allows the system 1 to display variations in metabolic activity immediately as they occur, which facilitates the study of transient responses of cells to environmental changes or to the addition of nutrients or drugs. The implementation of digital signal-processing algorithms reduces noise influence and ensures stable operation under varying measurement conditions.

A communication interface 9 is provided, which is configured to transmit the respiration-related parameters or derived data to an external device 10, for example a computer or display unit, for further processing, visualization, or storage. The communication interface 9 allows integration of the system 1 into laboratory networks and synchronization with other instruments, thereby enabling comprehensive data analysis and correlation of metabolic information with other physiological parameters.

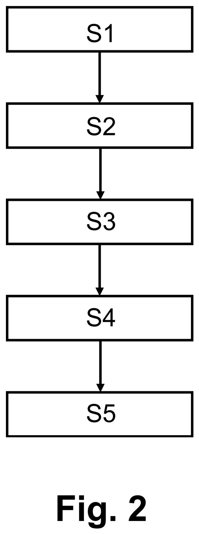

Fig. 2 illustrates a flow chart of a method for determining cellular respiration and nutrient metabolism of living cells or tissues that can be realized with the system 1 described above. In step S1, a biological sample 3 is received and maintained under controlled environmental conditions in the cell container 2. In step S2, gaseous components produced by the metabolic activity of the biological sample 3 accumulate in the gas volume 4 in fluid communication with the cell container 2. In step S3, the gas volume 4 is illuminated with light from the illumination source 6, and the detector arrangement 7 detects the absorption signals corresponding to ¹²CO₂ and ¹³CO₂. In step S4, the electronic control and evaluation unit 8 acquires and processes the absorption data to determine temporal variations of CO₂ concentration and isotopic composition. In step S5, respiration-related parameters of the biological sample 3 are output and transmitted via the communication interface 9 to the external device 10 for display, storage, or further analysis.

Through this sequence of steps, the method enables continuous and real-time monitoring of metabolic gas exchange under physiological conditions, providing quantitative and isotopically resolved information on cellular respiration and nutrient utilization.

### List of reference signs

- 1: system
- 2: cell container
- 3: biological sample
- 4: gas volume
- 5: optical detection unit
- 6: illumination source
- 7: detector arrangement
- 8: electronic control and evaluation unit
- 9: communication interface
- 10: external device
- 11: optical path
- 12: printed circuit board
- 13: sealing
- 14: off-axis parabolic mirrors

## Claims

1. A system (1) for determining cellular respiration and nutrient metabolism of living cells or tissues, the system comprising:
a cell container (2) configured to receive and maintain a biological sample (3),
a gas volume (4) in fluid communication with the cell container (2), the gas volume (4) containing gaseous components produced by metabolic activity of the biological sample (3),
an optical detection unit (5) configured to detect a target gas species in the gas volume by wavelength-selective absorption spectroscopy, the optical detection unit being further configured to distinguish between different isotopic species of the target gas species, the optical detection unit (5) comprising an illumination source (6) and detector arrangement (7) optically coupled to the optical detection unit (6) and configured for measurement of absorption signals corresponding to different isotopes of the target gas species, and
an electronic control and evaluation unit (8) operatively connected to the optical detection unit (7), the electronic control and evaluation unit (8) being configured to acquire and process absorption data, determine absolute values and/or temporal variations of the target gas species concentration and isotopic composition, and output respiration-related parameters of the biological sample (3).

2. The system (1) according to claim 1, wherein the electronic control and evaluation unit (8) is configured to process absorption data in real time.

3. The system (1) according to claim 1 or 2, wherein the illumination source (6) and the detector arrangement (7) are configured for non-dispersive infrared measurement of absorption signals corresponding to different isotopes of the target gas species.

4. The system (1) according to any of the preceding claims, wherein the optical detection unit (5) comprises at least one optical path (11) having an optical path length adapted for detection of target gas species concentrations in the range of less than 1 %, preferably less than 0.1 %.

5. The system (1) according to any of the preceding claims, wherein the illumination source (6) comprises a modulated infrared light-emitting diode or laser diode, and the detector arrangement (7) comprises a wavelength-selective photodetector or thermal detector.

6. The system (1) according to any of the preceding claims, wherein the optical detection unit (5) is configured to measure absorption signals at at least two discrete wavelength bands corresponding to the different isotopes of the target gas species absorption maxima.

7. The system (1) according to any of the preceding claims, wherein the electronic control and evaluation unit comprises a microcontroller or microprocessor configured to demodulate the detector signal and compute the ratio between the different isotopes of the target gas species concentrations.

8. The system (1) according to any of the preceding claims, wherein the gas volume (4) is hermetically sealed with respect to the ambient environment.

9. A method for determining cellular respiration and nutrient metabolism of living cells or tissues, the method comprising the steps of:
receiving and maintaining a biological sample (3) in a cell container (2),
allowing gaseous components produced by metabolic activity of the biological sample (3) to accumulate in a gas volume (4) in fluid communication with the cell container (2),
illuminating the gas volume (4) with light and detecting absorption signals corresponding to different isotopes of a target gas species,
acquiring and processing absorption data to determine temporal variations of the target gas species concentration and isotopic composition, and
outputting respiration-related parameters of the biological sample (3).

10. The method according to claim 9, wherein the absorption data are processed in real time to determine instantaneous respiration-related parameters of the biological sample.

11. The method according to claim 9 or 10, wherein non-dispersive infrared measurement of absorption signals corresponding to the different isotopes of the target gas species is used.
